# EUROPEAN PATENT APPLICATION

(11) **EP 3 470 504 A1**
(43) Date of publication of application: **17.04.2019**
(21) Application number: 18211035.3
(22) Date of filing: 02.12.2010
(51) Int. Cl.: C11D 1/72, C11D 1/88, C11D 1/92, C11D 3/386, C12N 9/20

(54) **SURFACTANTS THAT IMPROVE THE CLEANING OF LIPID-BASED STAINS TREATED WITH LIPASES**

(62) Divisional of application: 10787655.9
(71) Applicant: Danisco US Inc., Palo Alto, CA 94304 (US)
(72) Inventor: ADAMS, Christian, Palo Alto, CA California 94304 (US); COLLIER, Katherine, D., Palo Alto, CA California 94304 (US); PEPSIN, Michael, Jay, Palo Alto, CA California 94304 (US); SCHMIDT, Brian, Palo Alto, CA California 94304 (US)
(74) Representative: Mewburn Ellis LLP

(57) **Abstract**

Described are compositions and methods relating to the removal of oily stains from fabrics and other surfaces using a lipase in combination with a selected surfactant to mediate the release of fatty acids generated by the lipase. The compositions and methods have application in e.g. laundry cleaning and dishwashing

## Description

### PRIORITY

The present application claims priority to U.S. Provisional Application Serial No. 61/288,778, filed on December 21, 2009, which is hereby incorporated by reference in its entirety.

### TECHNICAL FIELD

The compositions and methods relate to the removal of oily stains from fabrics and other surfaces using a lipase in combination with a selected surfactant to mediate the release of fatty acids generated by the lipase. The compositions and methods have application in, *e.g*., laundry cleaning and dishwashing.

### BACKGROUND

Current laundry detergent and/or fabric care compositions include a complex combination of active ingredients such as surfactants, enzymes (protease, amylase, lipase, and/or cellulose), bleaching agents, a builder system, suds suppressors, soil-suspending agents, soil-release agents, optical brighteners, softening agents, dispersants, dye transfer inhibition compounds, abrasives, bactericides, and perfumes.

While far superior to cleaning products used only a few years ago, current laundry detergents do not provide a satisfactory solution for oily soil removal. Lipolytic enzymes, including lipases and cutinases, have been employed in detergent cleaning compositions for the removal of oily stains by hydrolyzing triglycerides to generate fatty acids. However, the resulting cleaning compositions are often little more (or no more) effective in removing oily stains than equivalent compositions that lack lipases or cutinases.

There exists a need for more efficient means for removing oily stains, particularly fatty acids, from fabrics.

### SUMMARY

The present compositions and methods relate to the removal of oily stains from fabrics and other surfaces using a lipase in combination with a selected surfactant to mediate the release of fatty acids generated by the lipase. The compositions and methods have numerous applications, particularly for laundry cleaning, dishwashing, and cleaning other hard surfaces.

In one aspect, cleaning composition for removing oily stains is provided, comprising: (a) a lipolytic enzyme for hydrolyzing fatty acid esters present in the oily stain to produce free fatty acids, and (b) a surfactant for solubilizing the free fatty acids in the cleaning composition, thereby releasing the free fatty acids from the stain, wherein the amount of release of fatty acids from the stain is greater than that achieved using an equivalent composition lacking the surfactant.

In some embodiments, the stain is on a fabric. In some embodiments, the stain is on dishware. In some embodiments, the cleaning composition is a laundry detergent or a dishwashing detergent. In some embodiments, the cleaning composition is a single composition comprising the lipolytic enzyme and the surfactant. In some embodiments, the cleaning composition is a two-part composition, the first part comprising the lipolytic enzyme and second part comprising the surfactant, wherein the first part and the second part are combined prior to contacting the stain.

In some embodiments, the surfactant is a sugar-based non-ionic surfactant. In some embodiments, the surfactant is a maltopyranoside or a glucopyranoside. In some embodiments, the surfactant is a cyclic-maltopyranoside. In some embodiments, the sugar is maltose, glucose, or sucrose. In some embodiments, the sugar-based surfactant has an aliphatic portion comprising at least 4 carbons.

In some embodiments, the surfactant is a Triton or oxide non-ionic surfactant. In some embodiments, the surfactant is a zwitterionic surfactant. In some embodiments, the surfactant is a FOS-choline or sulfobetaine. In some embodiments, the surfactant has an aliphatic portion comprising at least 8 carbons.

In another aspect, a method for removing an oily stain from a surface is provided, comprising: contacting the surface with a lipolytic enzyme and a surfactant, hydrolyzing fatty acid esters present in the oily stain with the lipolytic enzyme to produce free fatty acids, and solubilizing the free fatty acids produced by the lipolytic enzyme with the surfactant, thereby removing the oily stain from the surface.

In some embodiments, the lipolytic enzyme and the surfactant are present in a single cleaning composition. In some embodiments, the lipolytic enzyme and the surfactant are present in different cleaning compositions that are combined prior to the contacting. In some embodiments, the lipolytic enzyme and the surfactant are present in different cleaning compositions that are combined upon the contacting. In some embodiments, the method further includes rinsing the surface.

In some embodiments, the surfactant is a sugar-based non-ionic surfactant. In some embodiments, the surfactant is a maltopyranoside, a glucopyranoside, or a cyclic-maltopyranoside. In some embodiments, the sugar is maltose, glucose, or sucrose.

In some embodiments, the surfactant is a Triton or oxide non-ionic surfactant. In some embodiments, the surfactant is a zwitterionic surfactant. In some embodiments, the surfactant is a FOS-choline or sulfobetaine.

In some embodiments, the surface is a fabric surface. In some embodiments, the surface is a dishware surface. In some embodiments, the surface is a hard surface.

These and other aspects of the present compositions and methods will be apparent from the following description and drawings.

### BRIEF DESCRIPTION OF THE DRAWINGS

Fig. 1 is a graph showing the release of fatty acids into solution in the presence of different anionic surfactants.
Fig. 2 is a graph showing the release of fatty acids into solution in the presence of bovine serum albumin used at a high concentration.
Fig. 3 is a graph showing the release of fatty acids into solution in the presence of different cationic surfactants.
Fig. 4 is a graph showing the release of fatty acids into solution in the presence of different maltopyranosides.
Fig. 5 is a graph showing the release of fatty acids into solution in the presence of different thiomaltopyranosides.
Fig. 6A is a graph showing the release of fatty acids into solution in the presence of different cyclic-maltopyranosides.
Fig. 6B is a graph showing the chain-length dependence of fatty acid release using sugar based surfactants.
Fig. 7 is a graph showing the release of fatty acids into solution in the presence of different glucopyranosides.
Fig. 8 is a graph showing the release of fatty acids into solution in the presence of sucrose monododecanoate and another glucopyranoside.
Fig. 9 is a graph showing the release of fatty acids into solution in the presence of different Tritons.
Fig. 10 is a graph showing the release of fatty acids into solution in the presence of different cholates.
Fig. 11 is a graph showing the release of fatty acids into solution in the presence of different anionic and non-ionic oxide surfactants.
Fig. 12 is a graph showing the release of fatty acids into solution in the presence of different FOS-cholines.
Fig. 13 is a graph showing the release of fatty acids into solution in the presence of different FOS-cholines derivatives.
Fig. 14 is a graph showing the release of fatty acids into solution in the presence of different Cyclo FOS surfactants.
Fig. 15 is a graph showing the release of fatty acids into solution in the presence of different sulfobetaines.
Fig. 16 is a graph showing the release of fatty acids into solution in the presence of different CHAPS surfactants.
Fig. 17 is a graph showing the release of fatty acids into solution following pretrement of bacon fat stained microswatches with surfactants.
Figure 18 is a graph showing the release of fatty acids into solution as measured by HPLC.
Figs. 19A-B show the structure of several maltopyranoside surfactants (19A) and the average hydrophilic-lipophilic balance (HLB) of various maltopyranoside surfactants (19B).
Fig. 19C shows the structure of several thiomaltopyranoside surfactants.
Figs. 20A-B show the structures of two maltopyranoside surfactants having aliphatic side groups. Figs. 20C shows the structure of cyclohexylmethyl-P-D maltosides.
Figs. 21A-C show the structures of different glycopyranosides (21A and 21B) and the average hydrophilic-lipophilic balance (HLB) of various glycopyranosides (21C).
Figs. 22A-B show the structures of polyethylene ethers (22A) and the average hydrophilic-lipophilic balance (HLB) of various polyethylene ethers (22B).
Fig. 23A and 23B show the structure and HLB, respectively, of Triton surfactants.
Fig. 24 shows the structure of Tween surfactants.
Fig. 25A-B show the structures of sucrose monododecanoate (25A) and methyl-6-O-(N-heptylcarbamoyl)-α-D-glucopyranoside (ANAMEG 7; 25B) surfactants .
Fig. 26 shows the structure of MEGA surfactants.
Fig. 27 shows the structure of sodium cholates.
Fig. 28 shows the structure of triethyl ammonium chlorides.
Figs. 29A-I show the structures of different zwitterionic surfactants.
Figs. 30A-B show the structure of additional anionic and zwitterionic surfactants.

### DETAILED DESCRIPTION

### I. Definitions

Prior to describing the present compositions and methods the following terms are defined for clarity. Terms and abbreviations not defined should be accorded their ordinary meaning as used in the art:

As used herein, the term "fatty acid" refers to a carboxylic acid derived from or contained in an animal or vegetable fat or oil. Fatty acids are composed of a chain of alkyl groups containing from 4-22 carbon atoms and characterized by a terminal carboxyl group - COOH. Fatty acids may be saturated or unsaturated, and solid, semisolid, or liquid.

As used herein, the term "triglyceride" refers to any naturally occurring ester of a fatty acid and glycerol. Triglycerides are the chief constituents of fats and oils. The have the general formula of CH₂(OOCR₁)CH(OOCR₂)CH₂(OOCR₃), where R₁, R₂, and R₃ are usually of different chain length.

As used herein, the term "surfactant" refers to any compound generally recognized in the art as having surface active qualities. Surfactants generally include anionic, cationic, nonionic, and zwitterionic compounds, which are further described, herein.

As used herein, a "lipolytic enzyme" (E.C. 3.1.1) refers to any acyl-glycerol carboxylic ester hydrolase. Lipolytic enzymes include lipases (triacylglycerol acylhydrolases, E.C. 3.1.1.3) and cutinases (E.C. 3.1.1.50). Activities of lipolytic enzymes include acyltransferase activity, esterase activity, transesterase activity, and lipase activity, which may be related reactions.

As used herein, the term "detergent composition" refers to a mixture which is intended for use in a wash medium for the laundering of soiled fabrics, dished, or other surfaces. Detergent compositions in general contain surfactants, hydrolytic enzymes, builders, bleaching agents, bleach activators, bluing agents, fluorescent dyes, caking inhibitors, masking agents, antioxidants, and/or solubilizers.

As used herein, the term "dishwashing composition" refers to a mixture which is intended for use in a wash medium for washing or cleaning hard surfaces such as dishes (*i.e.,* plates, bowls, forks, knives, and other dishware). Dishwashing compositions include manual dishwashing compositions and automatic dishwashing compositions.

As used herein, the term "laundry cleaning composition" refers to a mixture which is intended for use in a wash medium for washing or cleaning fabrics.

As used herein, "dextrins" refer to short chain polymers of glucose (*e.g.,* 2 to 10 units).

As used herein, the term "oligosaccharide" refers to a compound having 2 to 10 monosaccharide units joined in glycosidic linkages. Such short chain polymers of simple sugars include dextrins.

As used herein, the terms "contacting" and "exposing" refer to placing a surfactant and lipolytic enzyme in sufficient proximity an oily stain or oily soil to enable the enzyme and surfactant to at least partially decrease the amount of the stain or soil by producing fatty acids that are solubilized in the surfactant. Contacting may occur in a washing machine, a sink, on a body surface, etc.

As used herein, a "sugar-based surfactant" is a molecule having surface active properties and comprising at least one carbohydrate functional group, or a derivative, thereof. Exemplary sugar-based surfactants are maltopyranosides, thiomaltopyransodies, glucopyranosides, and their derivatives.

As used herein, the singular terms "a," "an," and "the" includes the plural unless the context clearly indicates otherwise. Thus, for example, reference to a composition containing "a compound" includes a mixture of two or more compounds. The term "or" generally means "and/or," unless the content clearly dictates otherwise.

Headings are provided for convenience, and a description provided under one heading may apply equally to other parts of the disclosure. All recited species and ranges can be expressly included or excluded by suitable language or provisos.

Numeric ranges are inclusive of the numbers defining the range. Where a range of values is provided, it is understood that each intervening value between the upper and lower limits of that range is also specifically disclosed, to a tenth of the unit of the lower limit (unless the context clearly dictates otherwise). The upper and lower limits of smaller ranges may independently be included or excluded in the range.

All patents, patent applications, articles and publications mentioned herein, both *supra* and *infra,* are hereby expressly incorporated herein by reference.

### II. Introduction

Lipases can be added to cleaning compositions to remove lipid-based stains from fabric. It is generally thought that lipases hydrolyze triglycerides present in the stains to fatty acids, which are then released from the fabric into a wash solution. However, observations made in support of the present compositions and methods suggest that the fatty acids produce by the lipases may, in fact, be more difficult to remove from fabrics than triglycerides. This may account for the limit success of lipases-containing cleaning compositions in remove some oily stains.

To enhance the ability of lipases to affect the removal of lipid stains from fabrics, a series of experiments were performed to test the ability of surfactants to remove fatty acids produced by the hydrolysis of triglycerides by a lipase. Surprisingly, of the numerous surfactants tested, only a selected surfactant were effective in mediating the release of fatty acids from fabric, suggesting that the selection of a suitable surfactant is not a straightforward matter.

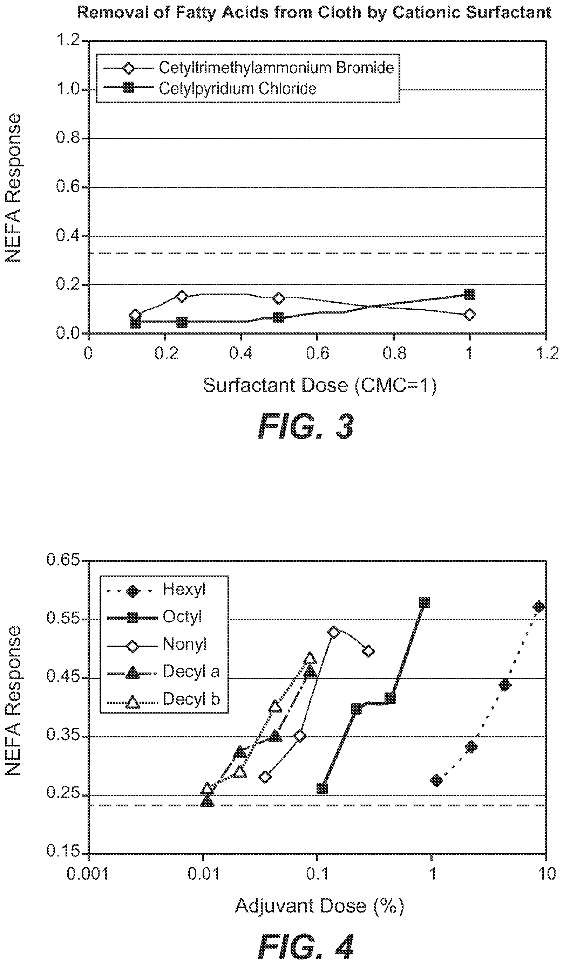

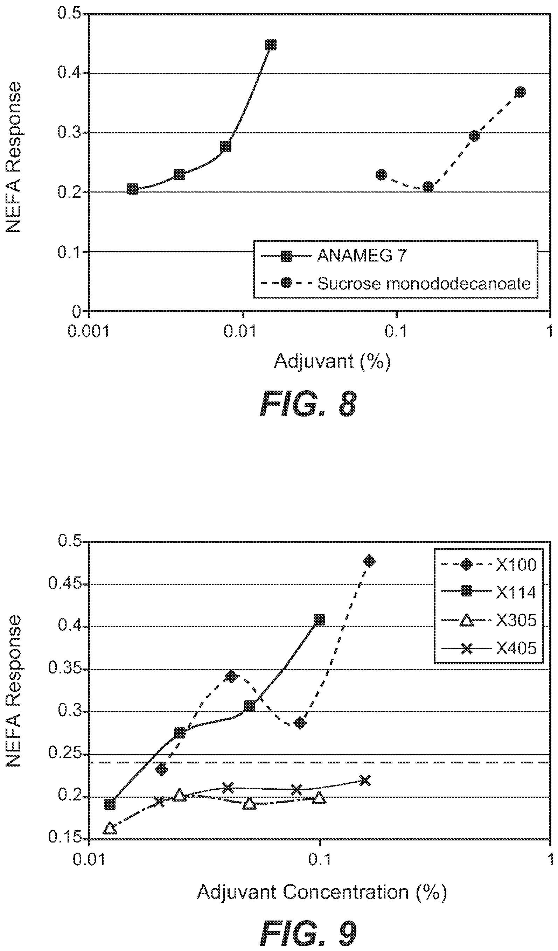

Of the nonionic surfactants, sugar-based surfactants were particularly effective at removing fatty acids from fabric swatches. Sugar-based surfactants having a long-chain length were more effective than short and branched-chain sugar-based surfactants (Figs. 4-8, 17 and 18). In some cases, maltose and sucrose-based surfactants were more effective than glucose-based surfactants. Among the non-ionic surfactants, certain Tritons and oxides were also effective (Fig. 9).

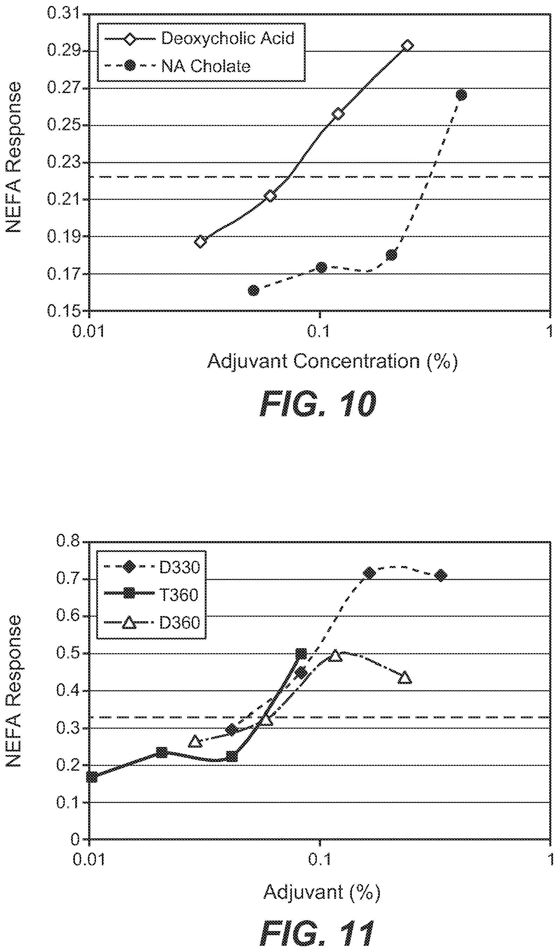

With respect to anionic surfactants, cholates and sarcosines (e.g., Fig. 10) were able to remove fatty acids from fabric swatches but only at concentrations higher than needed for some of the sugar-based surfactants.

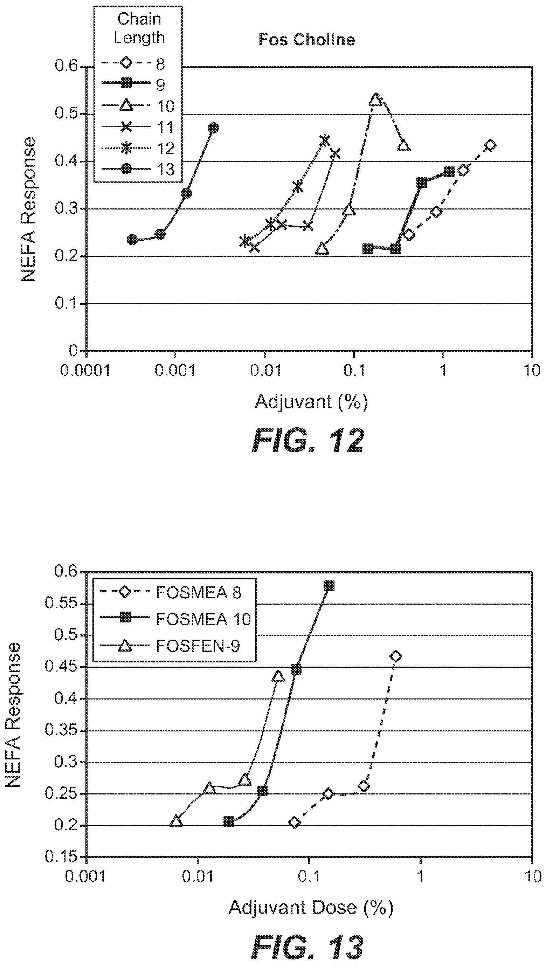

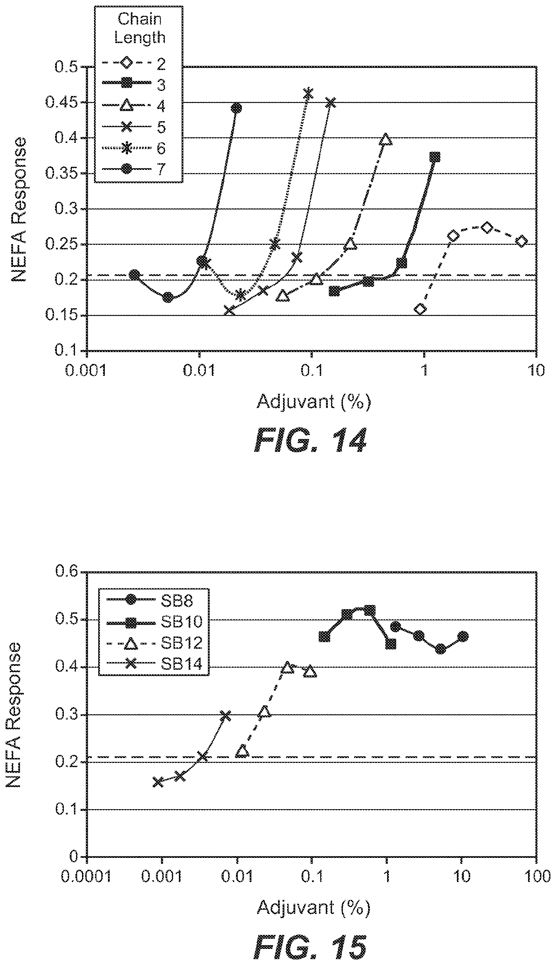

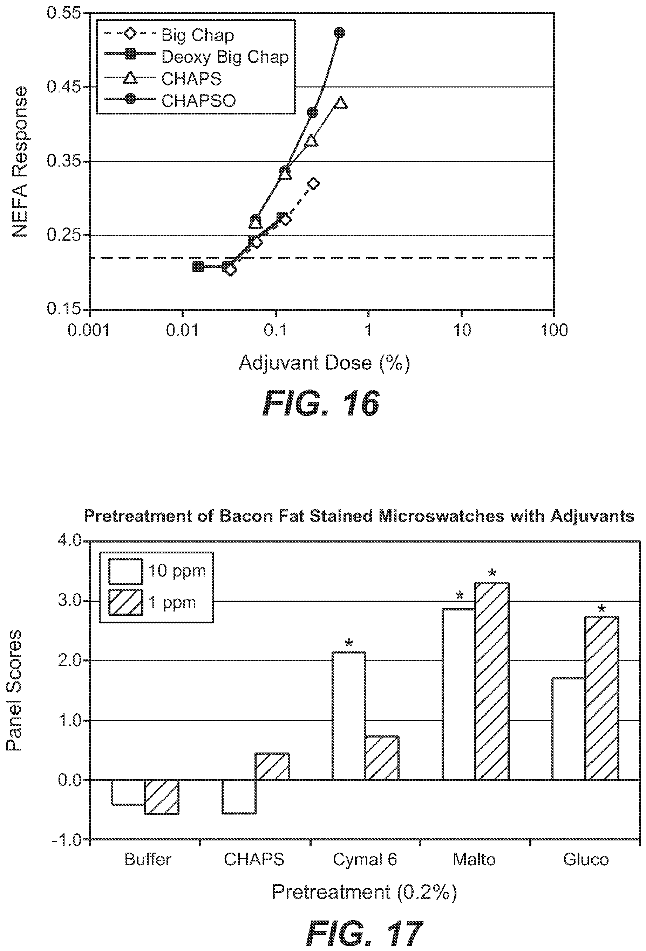

Several zwitterionic surfactants were effective in removing fatty acids from fabric, including longer chain-length FOS-cholines and variants, thereof (Figs. 12-14). The sulfobetaines were also effective (Fig. 15). The oxides (Fig. 11) and CHAPS (Fig. 16) based surfactants were effective but only at higher doses than the sulfobetaines.

Generally, the most effective surfactants had a relatively small hydrophilic portion with no net charge. The preferred hydrophobic portions were linear, saturated, and/or included an aliphatic hydrophobic portion. The best surfactants tended to be sugar-based compounds and zwitterionic compounds. Exemplary surfactants and method for their use are to be described.

### III. Compositions for removing oily stains

The present compositions include one or more adjuvants (*i.e.,* surfactants) and one or more lipolytic enzymes. In some embodiments, the adjuvant and lipolytic enzyme are present in a single composition. In other embodiments, the adjuvant and lipolytic enzyme are present in separate compositions that are combined before contacting an oil stain on fabric, or combined on the oil stain. Components of the present compositions are described, below.

### A. Adjuvants

The present cleaning compositions include one or more adjuvants (surfactants) for use in combination with a lypolytic enzyme. Suitable adjuvants have a relatively small hydrophilic portion with no net charge and hydrophobic portion that is linear or saturated. In some embodiments, the hydrophobic portion includes at least, six, seven, eight, or nine adjacent aliphatic carbons. In some embodiments, the hydrophobic portion is cyclic. In some embodiments, the hydrophobic portion is not branched. The best surfactants tended to be sugar-based compounds and zwitterionic compounds.

Suitable sugar-based surfactants include maltopyranosides, thiomaltopyransodies, glucopyranosides, and their derivatives. Maltose-based surfactants were generally more effective than glucose-based surfactants. Preferred sugar-based surfactants have a hydrophobic tail chain length of at least 4, at least 5, at least 6, and even at least 7 carbons. The tail should generally be aliphatic and may be cyclic. The tail should be unbranched, although it is likely that some branching is acceptable with sufficient chain length.

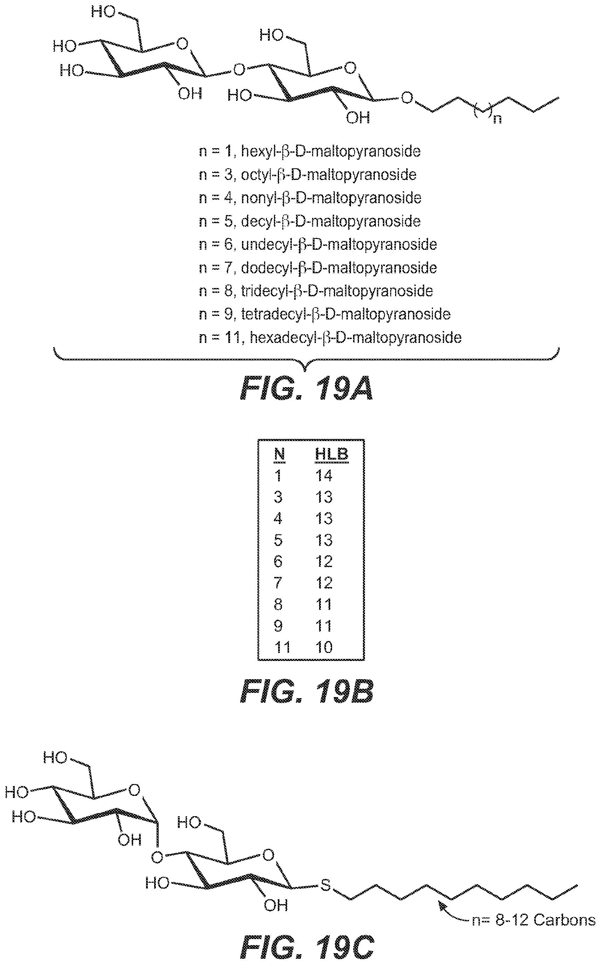

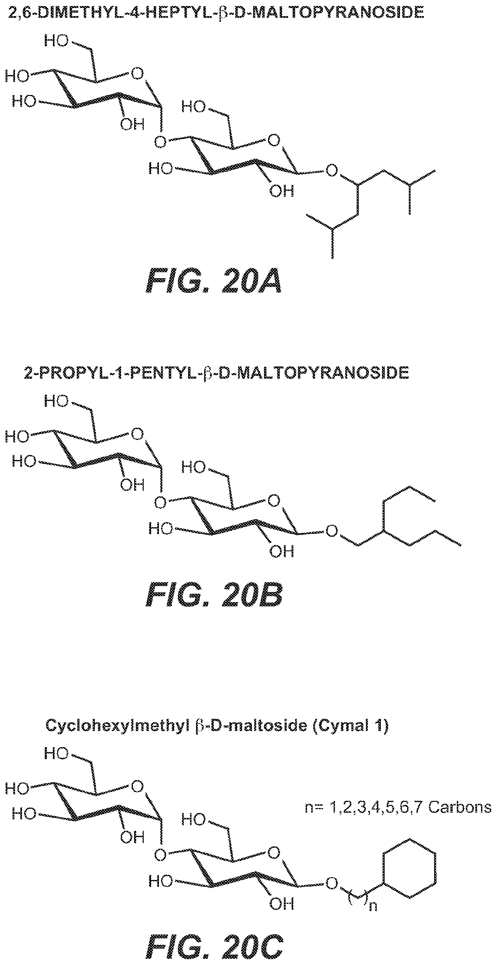

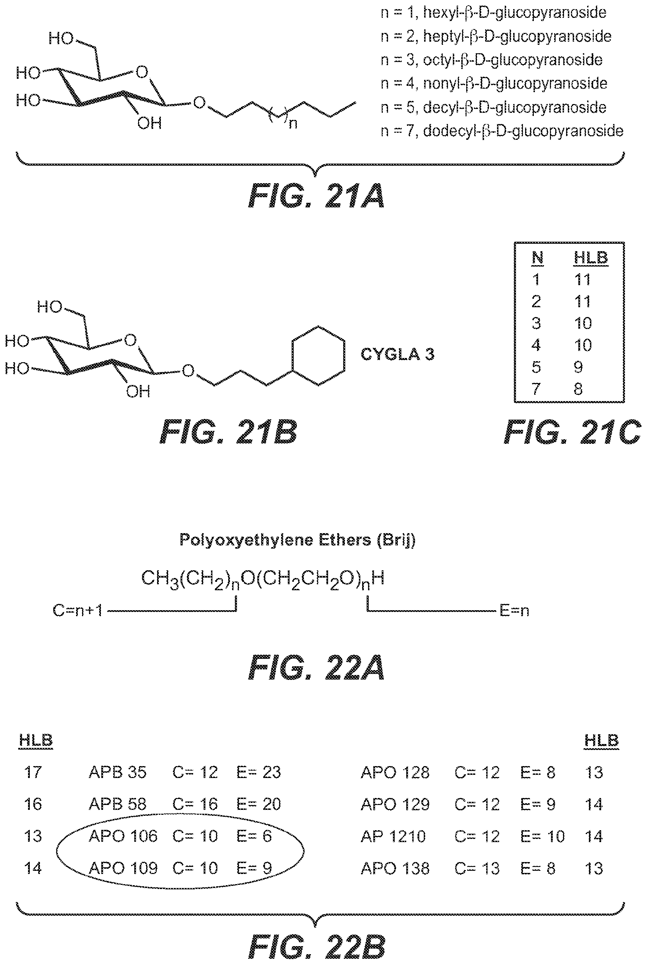

Particular examples of sugar-based surfactants are nonyl-P-D-maltopyranoside, decyl-β-D-maltopyranoside, undecyl-P-D-maltopyranoside, dodecyl-β-D-maltopyranoside, tridecyl-β-D-maltopyranoside, tetradecyl-β-D-maltopyranoside, hexaecyl-β-D-maltopyranoside, and the like, 2,6-dimethyl-4-heptyl-β-D-maltopyranoside, 2-propyl-1-pentyl-β-D-maltopyranoside, nonyl-P-D-glucopyranoside, nonyl-P-D-glucopyranoside, decyl-β-D-glucopyranoside, dodecyl-P-D-glucopyranoside, sucrose monododecanoate, certain cyclohexylalkyl-β-D-maltosides (*e.g.,* the CYMAL®s and CYGLAs), and the MEGA™ surfactants. The structures of many of these surfactants is shown in Figs. 19-21.

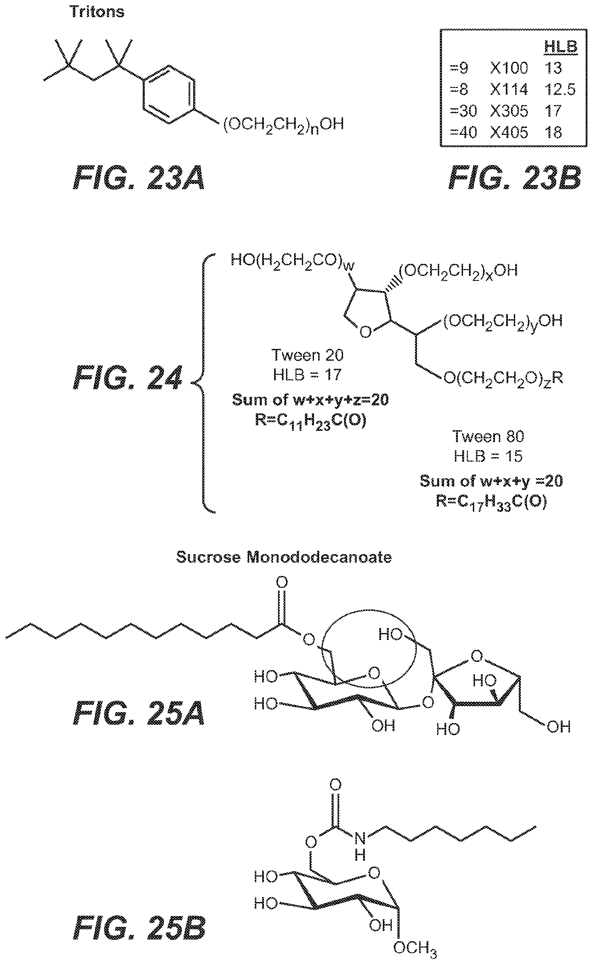

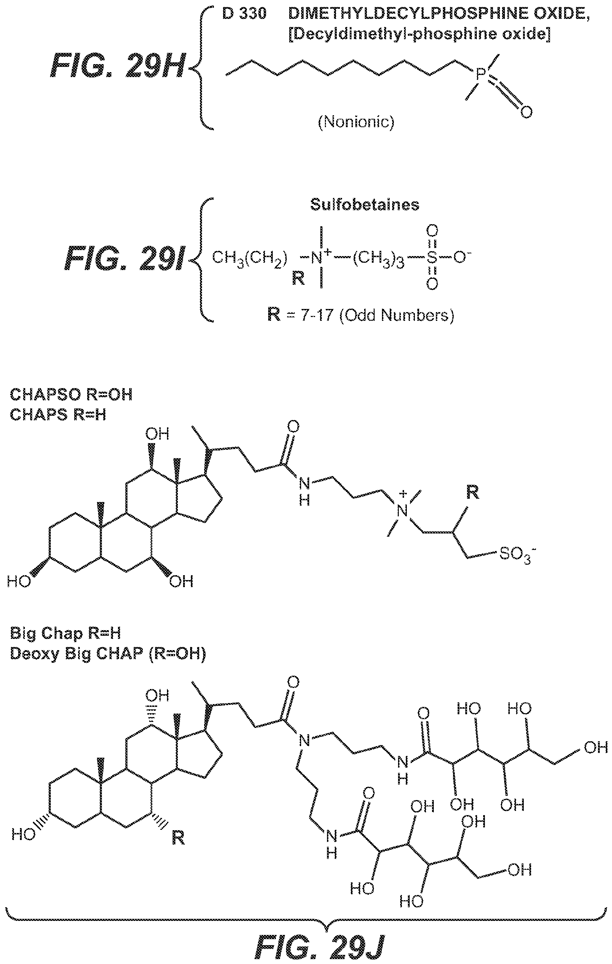

The adjuvant may be a non-sugar, non-ionic surfactant. Exemplary surfactants are Tritons with an ethoxylate repeat of nine or less. Particular Tritons are ANAPOE®-X-100 and ANAPOE®-X-114. The structure of some of these surfactants is shown in Fig. 24. In some embodiments, the adjuvant is a non-ionic phosphine oxide surfactant, having a hydrophobic tail of at least about 9 carbons. Exemplary surfactants are dimethyldecylphoshine oxide and dimethyldodecylphoshine oxide. The structure of some of these surfactants is shown in Fig. 29H.

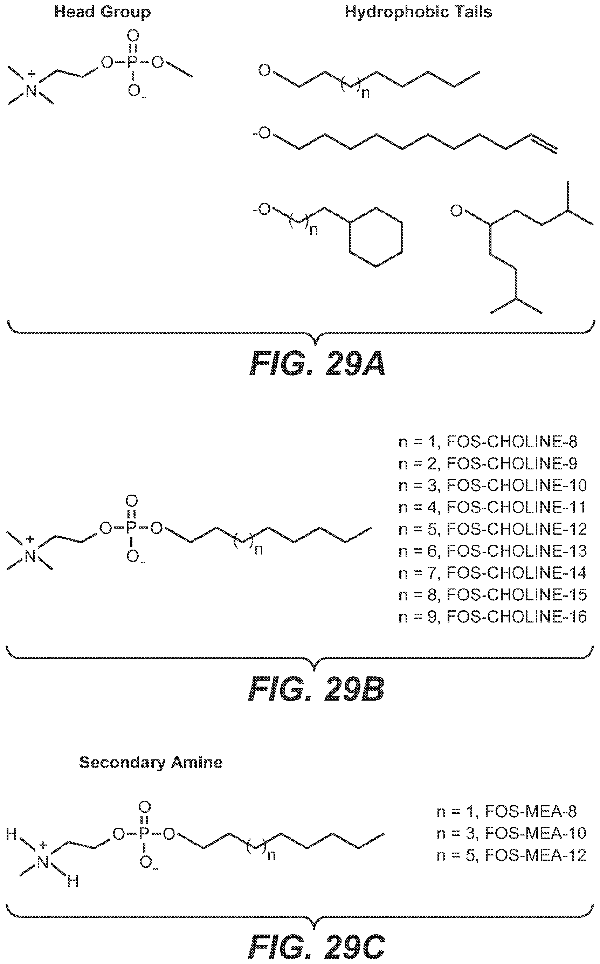

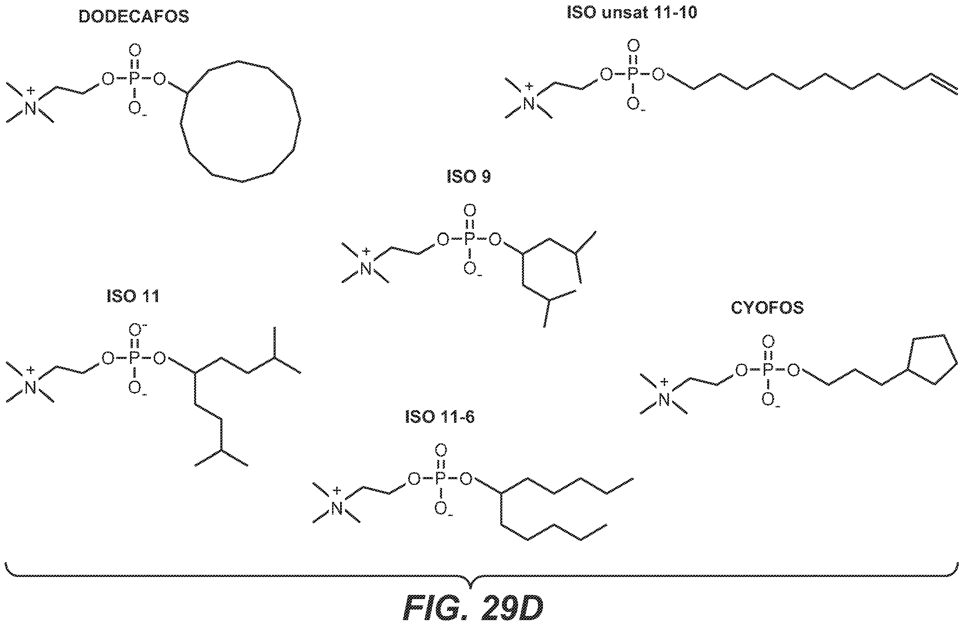

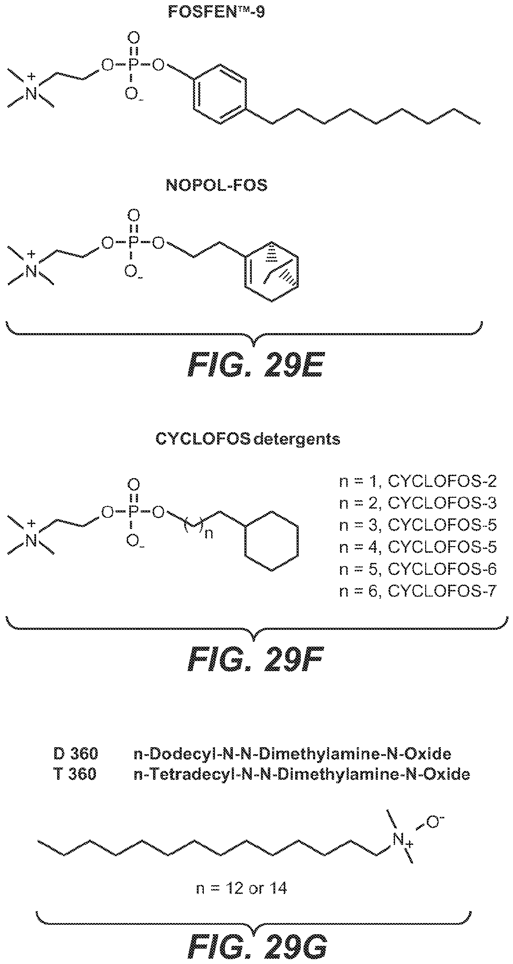

The adjuvant may be a zwitterionic surfactant, such as a FOS-choline, as shown in Fig. 29A-F. In some embodiments, the FOS-choline has a hydrophobic tail with a chain length of 12 or greater. The hydrophobic tail may be saturated and unsaturated and may be cyclic. Exemplary FOS-choline surfactants are FOS-CHOLINE®-12, FOS-CHOLINE®-13, FOS-CHOLINE®-14, FOS-CHOLINE®-15, FOS-CHOLINE®-16 (Fig. 29B), FOS-MEA®-12 (Fig. 29C), DODECAFOS, ISO unsat 11-10, ISO 11-6, CYOFO (Fig. 29D), NOPOL-FOS (Fig. 29E), CYCLOFOS® (CYMAL®)-5, -6. -7, -8, etc. (Fig 29F), and the like.

In some cases, the adjuvant is a sulfobetaine zwitterionic surfactant. Preferred sulfobetaine surfactants have a hydrophobic tail having at least 12 carbons, *e.g*., ANZERGENT® 3-12 and ANZERGENT® 3-14 (Fig. 29I). The zwitterionic oxides (Fig. 29G) and CHAPS (Fig. 29I)-based surfactants were also effective but only at higher doses than the sulfobetaines.

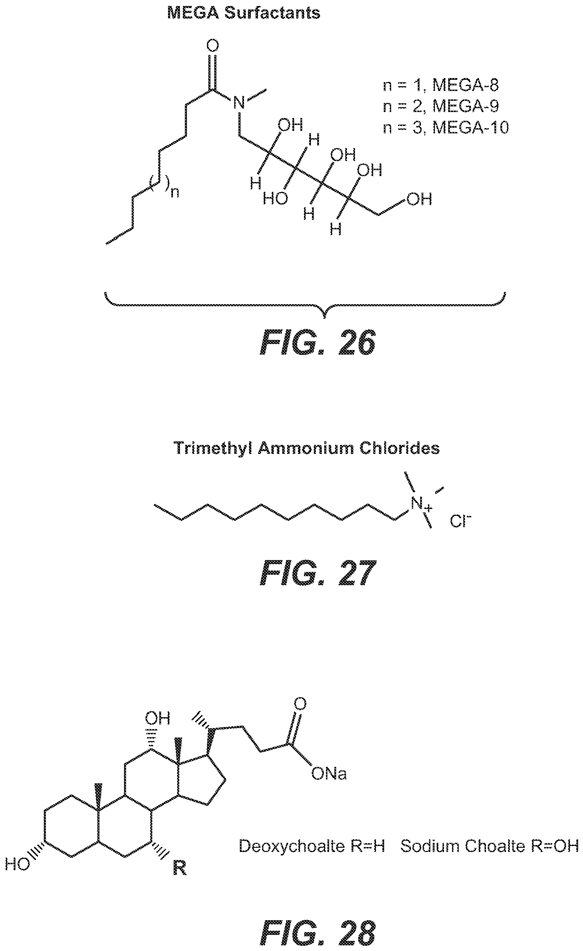

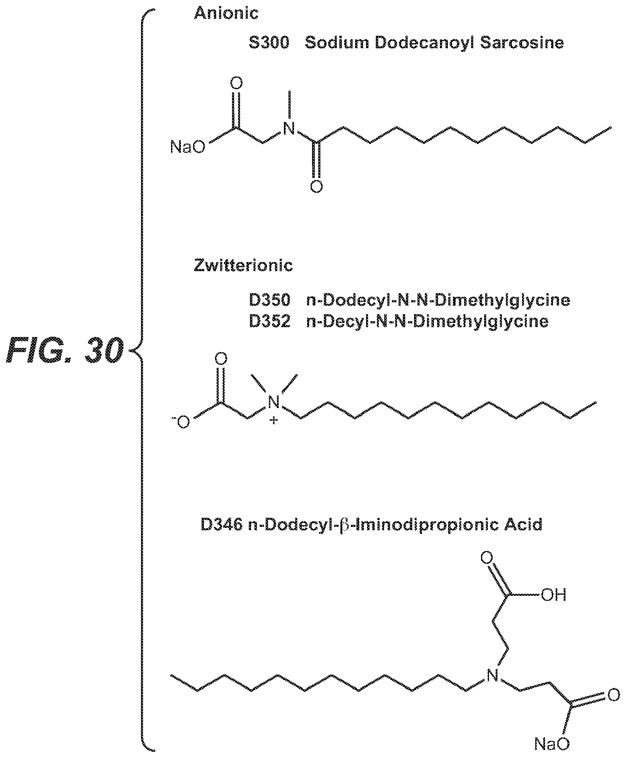

In some cases, the adjuvant may also be an anionic detergent, for example, a sarcosine (as shown in Fig. 30). Preferred sarcosines have a hydrophobic tail having at least 10 carbons. In some cases, the adjuvant may also be deoxycholate (as shown in Fig. 27).

The adjuvant may be present in a composition in an amount of at least .001%, at least 0.005%, at least 0.01%, at least 0.05%, at least 0.1%, or more, or at least 0.01 ppm, at least 0.05 ppm, at least 0.1 ppm, at least 0.5 ppm, at least 1 ppm, at least 5 ppm, at least 10 ppm, or more. In some cases, the adjuvant may be present in a preselected range, *e.g*., about 0.001-0.01%, about 0.01-0.1%, about 0.1-1%, or about 0.01-1 ppm, about 0.1-1 ppm, or about 1-10 ppm. In some cases, optimum activity is observed over a range, above and below which activity is reduced.

### B. Lipolytic enzymes

The present compositions include one or more lipolytic enzymes for use in combination with one or more adjuvants. Lipases include wild-type (*i.e.,* naturally-occurring) lipases and variant lipases, including fragments, having lipase activity. Extracellular lipases (E.C. 3.1.1.3) are produced by a wide variety of microorganisms such as fungi. Exemplary lipases are described in U.S. Patent Nos. US3950277, US6017866, US5990069, US5352594, US5445949, US5278066, US7511005, US5427936, US7781200, US7666630, US7396657, US7271139, US7226770, US7157263, US6939702, US6686189, US6624129, US6432898, US6156552, US6074863, US6020180, US5892013, US5869438, US5846801, US5830736, US5827718, US5766912, and US5763383; European Patent Nos. EP1625202, EP0528828, EP0468102, EP1625208, and EP0652946; and International Patent Nos. WO2010065451 and WO2010065455. Lipases may be obtained from such diverse microorganisms as *Pseudomonas, Aspergillus, Pneumococcus, Streptomyces, Staphylococcus, Corynebacterium, Mycobacterium, Mycotorula, Bacillus, Fusarium, Acinetobacter, Thermobifida, Magnaporthe, Geobacillus,* and *Sclerotinia.* Exemplary lipases can be obtained from *Streptomyces* spp., *e.g*., *Streptomyces rimosus, Streptomyces coelicolor, Streptomyces natalensis,* and *Streptomyces griseus; Corynebacterium* spp., *e.g., Corynebacterium efficiens, Pseudomonas* spp., *e.g*., *Pseudomonas aeruginosa, Pseudomonas pseudoalcaligenes, Pseudomonas plantarii, Pseudomonas mendocina,* and *Pseudomonas stutzeri; Fusarium* spp., *e.g*., *Fusarium solanii; Acinetobacter* spp., *Acinetobacter calcoaceticus; Thermobifida* spp., *e.g*., *Thermobifidafusca; Magnaporthe* spp., *e.g*., *Magnaporthe grisea; Geobacillus* spp., *e.g., Geobacillus stearothermophilus; Bacillus* spp., *e.g*., *Bacillus subtilis* and *Bacillus pumilus; Mycobacterium* spp., *e.g*., *Mycobacterium tuberculosis; Mycotorula* spp., *e.g*., *Mycotorula lipolytica;* or variants or homologues thereof.

Examples of the use of lipases in detergent compositions are found in. *e.g*., EP 463100 (*Pseudomonas alcaligenes*), EP 0218272 (*Pseudomonas pseudoalcaligenes*), EP 0214761 (*Pseudomonas cepacia*), EP 0258068 (*Thermomyces*), EP 206390 (*Pseudomonas chromobacter, Pseudomonas fluorescens, Pseudomonas fragi, Pseudomonas nitroreducens, Pseudomonas gladioli,* and *Chromobacter viscosum*), EP 0652946 (a lipase variant), EP 0 130 064 (*Fusarium oxysporum,* WO 90/09446 (*Fusarium solanii* var. *pisi*), and U.S. Patent No. 5,990,069 (*Fusarium solanii*). Any of these lipases are expected to be suitable for use as described, herein.

The exemplary lipase was a variant *Pseudomonas alcaligenes* lipase that includes the substitution M21L. This lipase is available as LIPOMAX™ (Danisco U.S. Inc, Genencor Division, Palo Alto, CA, USA). Wild type *Pseudomonas alcaligenes* lipase and pother variants are expected to produce similar results.

Cutinases are lipolytic enzymes capable of hydrolyzing the substrate cutin, although the activity of cutinases is typically more general, making the enzymes suitable for use in place of lipases. Cutinases expected to be suitable as described include wild-type (*i.e.,* naturally-occurring) lipases and variant lipases, including fragments, having lipase activity. Cutinases are produced by a wide variety of microorganisms such as fungi. Suitable cutinases for the present invention have been descried, for example, in Kolattukudy, P. E., "Lipases", Borgstrom, B. and Brockman, H.L. (eds.), Elsevier 1984, 471-504. The amino acid sequence and the crystal structure of a cutinase from *Fusarium solani pisi* have been described (Longhi, S. et al., J. Mol. Biol., 268 (4), 779-799 (1997)). The amino acid sequence of a cutinase from *Humicola insolens* has also been described (U.S. Patent No. US5827719).

Cutinases suitable for used as described include variants of cutinases from *Fusarium solani pisi* (WO 94/14963; WO 94/14964; WO 00/05389; and U.S. Patent No. US6960459. A cutinase obtained from *Pseudomonas mendocina* or a variant or homologue thereof may also be used.

### C. Carriers and formulations

In addition to one or more adjuvants (*i.e.,* surfactants) and/or one or more lipolytic enzymes, the present cleaning compositions may further include suitable carriers, buffers, polymers, additional hydrolytic and other enzymes, and other formulation ingredients.

Exemplary additional enzymes include proteases, carboxypeptidases, aminopeptidases, cellulases, xylanases, β-galactosidases, β-glucosidases, amylases, α-galactosidases, glucoamylases, α-glucosidases, carbohydrases, mannosidases, glycosyltransferases, laccases, catalases, peroxidases, oxidases, chitinases, cyclodextrin esterases, haloperoxidases, invertases, pectinolytic enzymes, peptidoglutaminases, phytases, polyphenoloxidases, transglutaminases, deoxyribonucleases, ribonucleases, and the like.

Suitable buffers are phosphate buffers, citrate buffers, acetate buffers, Tris, HEPES, MOPS, MES, and the like. The pH of the cleaning composition should be suitable for maintaining the activity of the lipolytic enzyme and for efficient surfactant activity, *e.g*., between about 4-10, between about 5-9, between about 6-8, and even between about 6.5-7.5.

Exemplary formulation ingredients include builders, bleaching agents, bleach activators, bluing agents, fluorescent dyes, caking inhibitors, masking agents, antioxidants, polymers, and solubilizers. Suitable detergent builders (or complexing agents) are zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (e.g., SKS-6 from Hoechst). Suitable polymers include carboxymethylcellulose (CMC), poly(vinylpyrrolidone) (PVP), polyethyleneglycol (PEG), poly(vinyl alcohol) (PVA), polycarboxylates such as polyacrylates, maleic/acrylic acid copolymers and lauryl methacrylate/acrylic acid copolymers.

The present cleaning compositions may in the form of a manual laundry detergent, and automatic laundry detergent, a manual dishwashing detergent, and automatic dishwashing detergent, a hand soap, a stain pretreatment composition, a shampoo, a facial cleaner, a general purpose cleaning composition, a car wash, and the like. As noted, the composition may be a one-part composition that includes both a lipolytic enzyme and a suitable surfactant to increase the release of fatty acids, or a two-part composition in which the lipolytic enzyme and a suitable surfactant are in different compositions. In the case of two-part composition, the parts may be combined and then contacted with an oily stain or combined on the oil stain. In some embodiments, the composition comprising the lipolytic enzyme is first contacted with the oily stain followed by the composition comprising the surfactant. In other embodiments, the composition comprising the surfactant is first contacted with the oily stain followed by the composition comprising the lipolytic enzyme.

While cleaning compositions are generally liquids, they can also be pastes, gels, granules, pellets, strips, bars, foams, and the like.

### IV. Methods for removing oily stains

In another aspect, methods for removing oily stains from fabrics are provided. The methods generally involve identifying fabrics having oily stains, contacting the fabrics with a cleaning composition comprising a lipase and a preselected selected adjuvant, and rinsing the fabric to remove the oily stain from the fabrics. In a related aspect, methods for removing oily stains from dishware or other hard surfaces are provided. The methods generally involve identifying dishware or other hard surfaces having oily stains, contacting the dishware or other hard surfaces with a cleaning composition comprising a lipase and a preselected selected adjuvant, and rinsing the dishware or other hard surfaces to remove the oily stain from the dishware.

In some embodiments, the lipase and the adjuvant are present together in a single composition. In some embodiments, the lipase and the adjuvant are separate in different compositions that are combined prior to contacting the fabric, dishware, or other hard surfaces, or mixed together on the fabric, dishware, or other hard surfaces. Therefore, application of the lipase and the adjuvant may be simultaneous of sequential.

In some embodiments, the contacting occurs in a wash pretreatment step, *i.e.,* prior to hand or machine-washing a fabric, dishware, or other hard surfaces. In some embodiments, the contacting occurs at the time of hand or machine-washing the fabric, dishware, or other hard surfaces. The contacting may occur as a result of mixing the present compositions with wash water, spraying, pouring, or dripping the composition on the fabric, dishware, or other hard surfaces, or applying the composition using an applicator.

The methods are effective for removing a variety of oil stains, or portions of oily stains, which typically include esters of fatty acids, such as triglycerides.

It will be appreciated that rinsing may occur some time after the washing, and that in some aspects the present method of cleaning is essentially complete following the contacting of the fabric with the composition.

Various modifications and variations of the described methods and system of the invention will be apparent to those skilled in the art without departing from the spirit and scope of the invention. Although the invention has been described in connection with specific preferred embodiments, it should be understood that the invention as claimed should not be unduly limited to such specific embodiments.

### EXAMPLES

The present compositions and methods are described in further detail in the following examples, which are intended to be illustrative rather than limiting in scope.

### Example 1: Effect of adjuvants on fatty acid removal from triglyceride-stained cotton swatches

In this example, the ability of adjuvants to remove fatty acids from triolein stained cotton microswatches was tested. Adjuvants from the JBSolution Detergent Test Kit (Catalog No. DK-101, Jena Bioscience GmbH, Jena Germany) containing stock solutions of 27 detergents ranging from ionic and non-ionic to zwitter-ionic detergents were used in this study.

### Surfactants

The surfactants tested include the following:

| Type | Examples |
|---|---|
| cationic | cetylpyridinium chloride, cetyltrimethylammonium bromide |
| non-ionic | Brij 35, Deoxy-BIGCHAP, HECAMEG, MEGA-8, MEGA-9, MEGA-10, n-Octyl-β-D-glucopyranoside, Pluronic F-68, sucrose monolaurate, Triton X-100, Triton X-114, Tween 20, Tween 80, Nonidet P40 |
| anionic | N-Lauroylsarcosin-sodium salt, lithiumdodecyl sulfate, sodium cholate, sodium deoxycholate, sodiumdodecylsulfate (SDS) |
| zwitterionic | CHAPS, CHAPSO, sulfobetaine SB 10, sulfobetaine SB 12, sulfobetaine SB 14, and sulfobetaine SB16 |

### Procedure

### A. Generation of fatty acids from triglyceride

0.25 inch diameter unsoiled cotton swatches (EMPA 221; Testfabrics, Inc. West Pittiston, PA) were used 96-well microtiter plates (MTP) (1 swatch per well, 5 swatches per test). 1 µl of 100% Triolein solution was then applied to each swatch. 150 µl of LIPOMAX™ (*Psuedomonas alcaligenes* lipase, Genencor International, Inc, Palo Alto, CA, USA) solution (0.67 ppm LIPOMAX™ in 50 mM HEPES pH 8.2 and 6 gpg water hardness) was then added to each well of the 96-well MTP which contained a triglyceride stained cotton swatch. The plates were incubated at 40°C for 1 hour with shaking at 500 RPM. After incubation, the supernatant was removed from the wells and the swatches were rinsed with 150 µl of 50 mM HEPES pH 8.2 buffer/6gpg water hardness. The rinse solution was removed from the wells and the swatches dried before use.

### B. Release of fatty acids from hydrolyzed triglycerides

Each surfactant was diluted to its critical micellar concentration (CMC) into 25 mM HEPES pH 8.2 buffer/6gpg water hardness. The surfactants were then serially diluted (1:1) three times. 100 µl of each of the four dilutions or each surfactants tested was added to wells of a 96-well plate, which contained the dried stained microswatches. All surfactants were dosed based on their CMC. Table 1 lists the surfactants and their CMCs. The plates were incubated at 40°C and subjected to shaking at 500 rpm for 30 minutes.

After incubation, the presence of fatty acids in the supernatant was detected using the HR Series NEFA-HR (2) reagent and kit (WAKO Diagnostics, Richmond, VA, USA) as recommended by the manufacturer. The NEFA kit measures non-esterified fatty acids. The cleaning performance of LIPOMAX™ plus adjuvants was compared to that of TIDE® 2X commercial detergent (Proctor & Gamble, Cincinnati, OH, USA). The extent of cleaning observed with TIDE® 2X detergent is shown as a horizontal dashed line on the Figures.

**Table 1. CMC of surfactants tested for fatty acid removal from triglyceride stained cotton swatches**

| Surfactant | | CMC (mM) |
|---|---|---|
| Cationic | Cetylpyridinium chloride | 1 |
| | Cetyltrimethylammonium bromide | 0.12 |
| Anionic | N-Lauroylsarcosin-Sodium salt | 13.7 |
| | Lithiumdodecyl sulfate | 8.7 |
| | Sodium deoxycholate | 10 |
| | Sodium dodecylsulfate | 8 |
| | Sodium cholate | 14 |

### Results

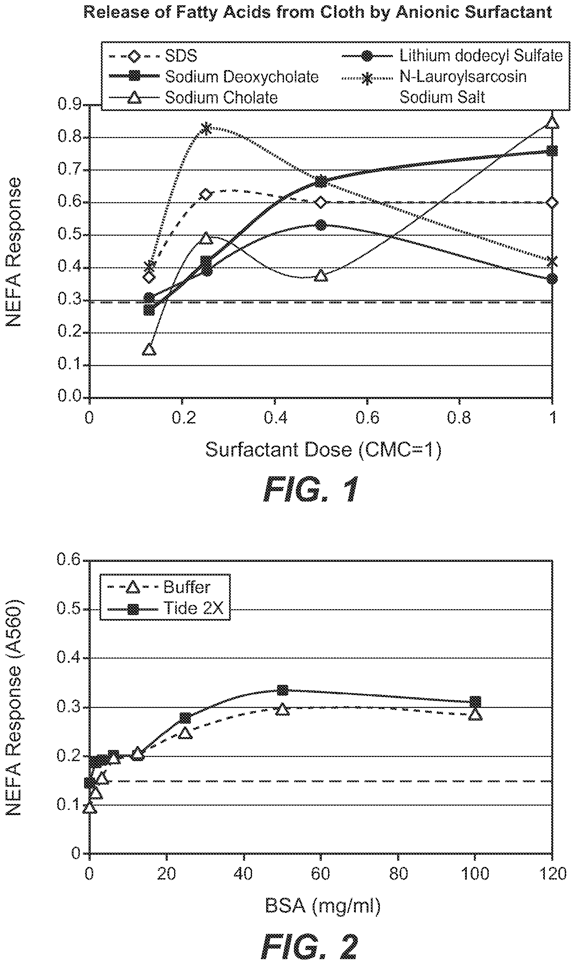

As shown in Fig. 1, several anionic surfactants were effective at removing fatty acids from triolein stained microswatches in combination with a lipase. At high concentrations, bovine serum albumin (BSA) was also effective at removing fatty acids from triolein stained microswatches in combination with a lipase (Fig. 2). Cationic surfactants were not very effective (Fig. 3).

### Example 2. Effect of adjuvants on fatty acid removal from bacon fat stained cotton microswatches

In this example, the ability of adjuvants to remove fatty acids from bacon fat-stained cotton microswatches was tested. Adjuvants from the Master Detergent Kit (DSOL-MK, Anatrace, Inc. Maumee, OH, USA), containing 100 different 10% solutions of a variety of nonionic, anionic, zwitterionic and a few catioinic surfactants, were used in this study. Some of the surfactants were members of "families," which included a variety of different hydrophobic chain lengths.

### Surfactants

The surfactants tested include the following:

| Type | Examples |
|---|---|
| non-ionic | sugar based: glucopyranosides, maltopyranosides, thiomaltopyransodies, 2,6-dimethyl-4-heptyl-β-D-maltopyranoside, 2-propyl-1-pentyl maltopyranoside, sucrose monododecanoate, ANAMEG® -7, MEGA-8; |
| | polyoxyethylene ethers: hexaethylene glycol monooctyl ether (C8E6), octaethylene glycol monododecyl ether (C12E8), pentaethylene glycol monodecyl ether (C10E5), tetraethylene glycol monooctyl ether (C8E4)), Tween (ANAPOE®-20 and ANAPOE®-80), and Triton (ANAPOE®-X-100, ANAPOE®-X-114, ANAPQE®-X-305, ANAPQE®-X-405) |
| cationic | decyltrimethylammonium chloride, dodecyltrimethylammonium chloride, hexadecyltrimethylammonium chloride, octadecyltrimethylammonium chloride, tetradecyltrimethylammonium chloride |
| anionic | deoxycholic acid, sodium salt, sodium cholate, sodium dodecanoyl sarcosine |
| zwitterionic | FOS choline surfactants: FOS-CHOLINE®-8, FOS-CHOLINE®-9, FOS-CHOLINE®-10, FOS-CHOLINE®-11, FOS-CHOLINE®-12, FOS-CHOLINE®-13, FOS-CHOLINE®-14, FOS-CHOLINE®-15, FOS-CHOLINE®-16, FOS-CHOLINE®-ISO-9, FOS-CHOLINE®-ISO-11, FOS-CHOLINE®-ISO-11-6U, FOS-CHOLINE®-UNSAT-11-10, FOS-MEA®-8, FOS-MEA®-10, FOSFEN™-9, NOPOL-FOS™, C-DODECAFOS™, CYCLOFOS™-2, CYCLOFOS™-3, CYCLOFOS™-4, CYCLOFOS™-5, CYCLOFOS™-6, CYCLOFOS™-7; |
| | Oxides: n-tetradecyl-N,N-dimethylamine-N-oxide (TDAO), n-dodecyl-N,N-dimethylamine-N-oxide (DDAO), dimethyldecylphosphine oxide; Sherpas-Polymeric Solubilization Aids: PMAL™-C8, PMAL™-C10; |
| | Sulfobetaines: ANZERGENT® 3-8, ANZERGENT® 3-10, ANZERGENT® 3-12, ANZERGENT® 3-14, Big CHAP, Big CHAP, deoxy, CHAPS, CHAPSO, n-Decyl-N,N-dimethylglycine, n-Dodecyl-N,N-dimethylglycine, n-dodecyl-β-iminodipropionic acid (monosodium salt) |

### Procedure

### A. Generation of fatty acids from bacon fat

0.25 inch diameter unsoiled cotton swatches (EMPA 221; Testfabrics, Inc. West Pittiston, PA) were used 96-well microtiter plates (MTP) (1 swatch per well, 5 swatches per test). 1 µl of bacon fat (obtained from frying Oscar Meyer Bacon, heated to 98°C) was then applied to each swatch. 150 µl of LIPOMAX™ (*Psuedomonas alcaligenes* lipase, Genencor International, Inc, Palo Alto, CA, USA) solution (0.67 ppm LIPOMAX™ in 50 mM HEPES pH 8.2 and 6 gpg water hardness) was then added to each well of the 96-well MTP which contained a bacon fat stained cotton swatch. The plates were incubated at 40°C for 1 hour with shaking at 500 RPM. After incubation, the supernatant was removed from the wells and the swatches were rinsed with 150 µl of 50 mM HEPES pH 8.2 buffer/6 gpg water hardness. The rinse solution was removed from the wells and the swatches dried before use.

### B. Release of the Fatty acids from hydrolyzed bacon fat

Each surfactant was diluted to its critical micellar concentration (CMC) into 25 mM HEPES pH 8.2 buffer/6 gpg water hardness. The surfactants were then serially diluted (1:1) three times. 100 µl of each of the four dilutions or each surfactants tested was added to wells of a 96-well plate, which contained the dried stained microswatches. The plates were incubated at 40°C and subjected to shaking at 500 rpm for 30 minutes.

After incubation, the presence of fatty acids in the supernatant was detected using the HR Series NEFA-HR (2) reagent and kit (WAKO Diagnostics, Richmond, VA, USA) as recommended by the manufacturer. The NEFA kit measures non-esterified fatty acids. The cleaning performance of LIPOMAX™ plus adjuvants was compared to that of heat-inactivated TIDE® 2X commercial detergent (Proctor & Gamble, Cincinnati, OH, USA). The extent of cleaning observed with TIDE® 2X detergent is shown as a horizontal dashed line on the Figures.

### Results

### A. Non-ionic surfactants

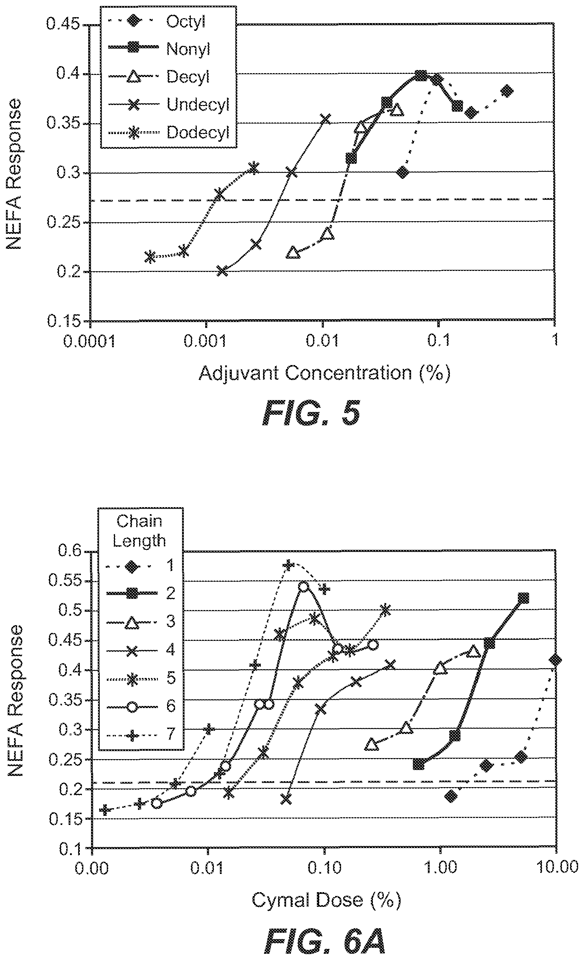

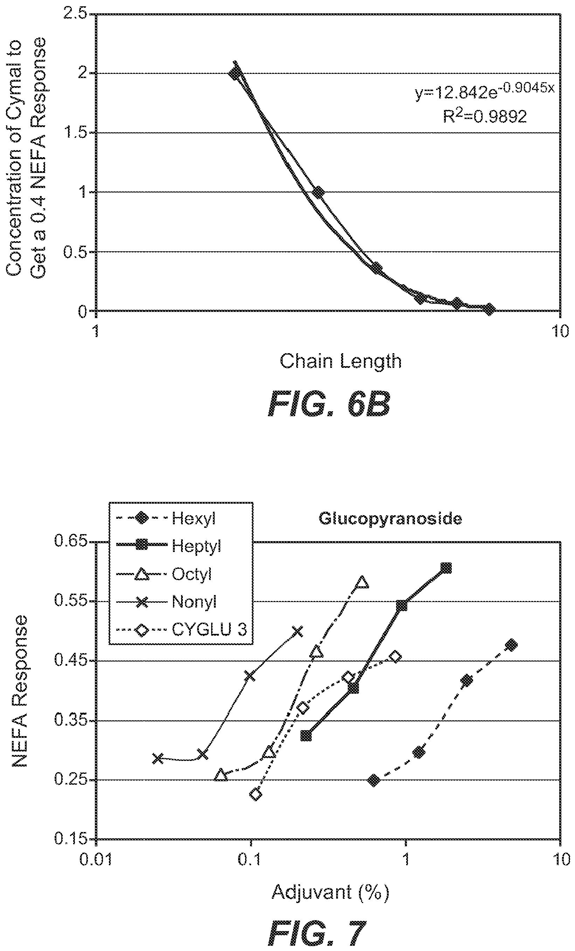

As shown in several different types of sugar-based surfactants were effective at removing fatty acids from bacon fat stained microswatches in combination with a lipase, for example, maltopyranosides (Fig. 4), thiomaltopyranosides (Fig. 5), cyclic-maltopyranosides (CYMAL®; Fig. 6A), and glucopyranosides (Fig. 7). Generally, longer-chain sugar-based surfactants were more effective at removing fatty acids in combination with a lipase than a short and/or branched-chain sugar-based surfactants. As a result, a lesser amount of a long-chain sugar-based surfactants is required to achieve the effect observed with a greater amount of a short-chain sugar-based surfactants. This point is illustrated in Fig. 6B for the cyclic-maltopyranosides. Note that about ten times more CYMAL® 2 is required than CYMAL® 7 to produce an equivalent release of fatty acids. Sucrose monododecanoate was also effective, while methyl-6-O-(N-heptylcarbamoyl)-α-D-glucopyranoside (*i.e.,* ANAMEG®-7) was not (Fig. 8).

Tritons with short-chain hydrophilic tails were also effective at removing fatty acids from bacon fat stained microswatches (Fig. 9). As shown in Fig. 11, a nonionic oxide surfactant, *i.e.,* dimethyldecylphosphine oxide (D 330; shown in Fig. 29H) was also effective. B. Anionic surfactants

As shown in Fig. 10, deoxycholate (R=H) as moderately effective at removing fatty acids from bacon fat stained microswatches in combination with a lipase, while sodium (Na) cholate (R=OH) was less effective.

### C. Zwitterioinc surfactants

As a class, zwitterioinc surfactants were effective at removing fatty acids from bacon fat stained microswatches in combination with a lipase. For example, as shown in Fig. 11, both n-dodecyl-N-N-dimethylamine-N-oxide (D 360) and n-tetradecyl-N-N-dimethylamine-N-oxide (T 360) (shown in Fig. 29G) were effective.

FOS-choline surfactants have a phosphocholine headgroup but, unlike phospholipids, possess simple hydrophilic tails (Fig. 29A-F). FOS-Cholines were effective at removing fatty acids from bacon fat stained microswatches in combination with a lipase (Fig. 12). The most effective FOS-cholines had a chain length of 12 or greater. Saturated and unsaturated FOS cholines were both effective. FOS-choline derivatives were also effective (Fig. 13).

Cyclo-FOS surfactants combine the phosphocholine headgroup with an aliphatic tail containing a cyclhexyl group as present in the CYMAL® series of detergents (Figs. 29F). Cyclo-FOS surfactants were also effective at removing fatty acids from bacon fat stained in combination with a lipase (Fig. 14).

As shown in Figs. 15 and 16, respectively, sulfobetaines and CHAPS series zwitterionic surfactants were effective. The structures of these surfactants are shown in Figs. 29I and 29J, respectively. For CHAPS, R=H; for CHAPSO, R=OH; for Big Chap, R=H; and for deoxy Big CHAP, (R=OH).

### Example 3. Effect of pretreatment on triglyceride stain removal from microswatches

In this example, experiments were performed to test if pretreatment of triglyceride stains in a low hardness environment would lead to increased stain removal. Medium scoured 460-U cotton knit greige good microswatches (Test Fabrics, Inc. West Pittiston, PA) were spotted with 50 µl of olive oil (neat), bacon fat (obtained from frying Oscar Meyer Bacon), and lard (ConAgra Foods, Omaha, NE), which were all heated to 98°C.

Air-dried stained swatches were incubated with 1 ppm or 10 ppm LIPOMAX™ and 0.2% adjuvants (n-octyl-β-D-glucopyranoside, Cat # 494460 (Calbiochem), n-decyl-β-D-Maltopyranoside, Cat # 252718 (Calbiochem), cyclohexyl-n-hexyl-P-D-maltoside (CYMAL® 6), Cat# 239775 (Calbiochem), or CHAPS, (Anatrace)) in 50 ml Sarstedt tubes in a total volume of 12 ml containing 25 mM HEPES buffer pH 8.2 per tube for 2.5 hours at room temperature with rocking. The swatches were then washed in heat inactivated TIDE® 2X Cold Water Detergent (Procter & Gamble, Cincinati, OH, USA), which was heat inactivated for 1 hour at 40°C to kill endogenous enzymes.

After incubation, the swatches were rinsed in water, air dried, and scored visually for stain removal by a panel of four people blinded to the treatment protocol. The extent of cleaning was numerically scored by 7 panelists blinded to the treatment who performed a pair-preference test based on the ranking scheme shown below:

| Score | Description |
|---|---|
| 0 | There was no difference |
| + 1 or - 1 | There might be a difference |
| + 2 or - 2 | There was a difference |
| + 3 or - 3 | There was a big difference |
| + 4 or - 4 | There was a substantial difference |

All cleaning was compared to that observed with TIDE® 2X Cold Water Detergent, which includes both non-ionic and anionic surfactants. Thus, a positive (+) score was assigned only if the sample cloth cleaned using a test adjuvant appeared cleaner than the corresponding cloth cleaned with TIDE® 2X Cold Water Detergent without an added adjuvant. A negative score (-) was assigned if the TIDE® 2X Cold Water Detergent treated swatch appeared cleaner than the test swatch. The results are shown in Fig. 17. "*" denotes a 98% confidence level that the swatch is cleaner than the TIDE® 2X Cold Water Heat inactivated control. As described above, sugar-based surfactants (*i.e.,* cyclic-maltopyranosides, maltopyranosides, and glucoopyranosides) were most effective. Noncyclic maltopyranosides and glucoopyranosides appear to produce more effective cleaning activity at lower doses, and may have an optimal dose range above which cleaning activity diminishes.

### Example 4. Fatty acid removal by adjuvants from microswatches as measured by HPLC

In this example, fatty acid removal from stained microswatches in the presence of adjuvants was monitored by HPLC. Unsoiled cotton microswatches (Testfabrics, Inc. West Pittiston, PA) were placed in 96-well microtiter plates and spotted with 2.5 µL of neat oleic acid (unsaturated fatty acid) or 15 µL of a 5g/L solution of steric acid (saturated fatty acid) dissolved in 1:1 acetone:hexane solution. The swatches were air dried for 20 minutes and incubated in either buffer (50 mM phosphate buffer, pH 8), heat inactivated TIDE® 2X Cold Water Detergent (Procter & Gamble, Cincinati, OH, USA), or 0.4% octyl β-D-glucopyranoside (OPG) in buffer at 30°C for 1 hour with shaking. After incubation, 100 µL of the supernatant was diluted 10X in 1:1 acetone:hexane solution. 200 µL of diluted oleic acid sample or 800 µL of steric acid sample were dried to remove the organic phase in a speed vacuum centrifuge. The fatty acids were labeled as bromophenacyl ester derivatives and analyzed by HPLC as described below.

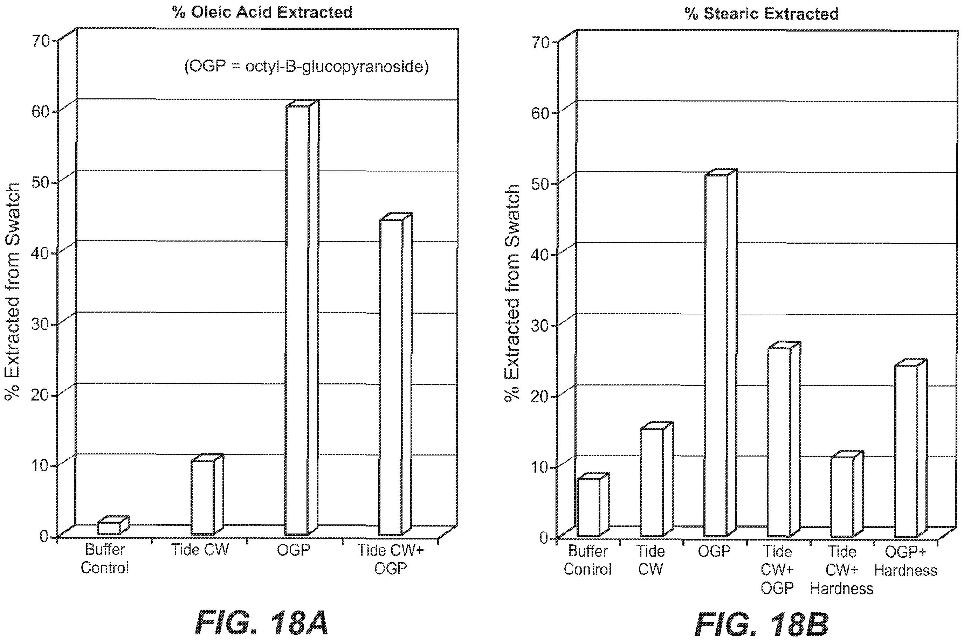

An Agilent 1100 (Hewlet Packard) HPLC was equipped with Ascentis C18 column, (Supelco). Fatty acids were eluted using a 60%-100% gradient of acetonitrile. The HPLC system was interfaced to a UV detector and fatty acids were detected at 242 nm. The results are shown in Fig. 18. The presence of the glucopyranoside (OPG) clearly improved the release of fatty acids and therefore, the cleaning activity, observed using the glucopyranoside. As indicated by the two bars on the far right of each panel, it is more difficult to remove fatty acids water hardness is increases.

The following numbered paragraphs (paras) contain further statements of various aspects of the present invention:
1. A cleaning composition for removing oily stains, comprising:
   a) a lipolytic enzyme for hydrolyzing fatty acid esters present in the oily stain to produce free fatty acids, and
   b) a surfactant for solubilizing the free fatty acids in the cleaning composition, thereby releasing the free fatty acids from the stain,
   wherein the amount of release of fatty acids from the stain is greater than that achieved using an equivalent composition lacking the surfactant.
2. The cleaning composition of para 1, wherein the cleaning composition is a laundry detergent or a dishwashing detergent.
3. The cleaning composition of para 1, wherein the cleaning composition is a single composition comprising the lipolytic enzyme and the surfactant.
4. The cleaning composition of para 1, wherein the cleaning composition is a two-part composition, the first part comprising the lipolytic enzyme and second part comprising the surfactant, wherein the first part and the second part are combined prior to contacting the stain.
5. The cleaning composition of any of the preceding paras, wherein the surfactant is a sugar-based non-ionic surfactant.
6. The cleaning composition of any of the preceding paras, wherein the surfactant is a maltopyranoside or a glucopyranoside.
7. The cleaning composition of any of the preceding paras, wherein the surfactant is a cyclic-maltopyranoside.
8. The cleaning composition of para 5, wherein the the sugar is maltose, glucose, or sucrose.
9. The cleaning composition of para 5, wherein the, the sugar-based surfactant has an aliphatic portion comprising at least 4 carbons.
10. The cleaning composition of any of paras 1-4, wherein the, the surfactant is a Triton or oxide non-ionic surfactant.
11. The cleaning composition of any of paras 1-4, wherein the surfactant is a zwitterionic surfactant.
12. The cleaning composition of any of paras 1-4, wherein the surfactant is a FOS-choline or sulfobetaine.
13. The cleaning composition of any of paras 1-4, wherein the the surfactant has an aliphatic portion comprising at least 8 carbons.
14. A method for removing an oily stain from a surface, comprising:
   contacting the surface with a lipolytic enzyme and a surfactant,
   hydrolyzing fatty acid esters present in the oily stain with the lipolytic enzyme to produce free fatty acids, and
   solubilizing the free fatty acids produced by the lipolytic enzyme with the surfactant,
   thereby removing the oily stain from the surface.
15. The method of para 14, wherein the lipolytic enzyme and the surfactant are present in a single cleaning composition.
16. The method of para 14, wherein lipolytic enzyme and the surfactant are present in different cleaning compositions that are combined prior to the contacting.
17. The method of para 14, wherein lipolytic enzyme and the surfactant are present in different cleaning compositions that are combined upon the contacting.
18. The method of any of paras 14-17, wherein the method further includes rinsing the surface.
19. The method of any of paras 14-18, wherein the surfactant is a sugar-based non-ionic surfactant.
20. The method of any of paras 14-19, wherein the surfactant is a maltopyranoside, a glucopyranoside, or a cyclic-maltopyranoside.
21. The method of para 19, wherein the sugar is maltose, glucose, or sucrose.
22. The method of any of paras 14-18, wherein the surfactant is a Triton or oxide non-ionic surfactant.
23. The method of any of paras 14-18, wherein the surfactant is a zwitterionic surfactant.
24. The method of any of paras 14-18, wherein the surfactant is a FOS-choline or sulfobetaine.
25. The method of any of paras 14-24, wherein the surface is a fabric surface.
26. The method of any of paras 14-24, wherein the surface is a dishware surface.
27. The method of any of paras 14-24, wherein the surface is a hard surface.

## Claims

1. A cleaning composition for removing oily stains, comprising:
a) a lipolytic enzyme for hydrolyzing fatty acid esters present in the oily stain to produce free fatty acids, and
b) a surfactant for solubilizing the free fatty acids in the cleaning composition, thereby releasing the free fatty acids from the stain,
wherein the amount of release of fatty acids from the stain is greater than that achieved using an equivalent composition lacking the surfactant.

2. The cleaning composition of claim 1, wherein the cleaning composition is a laundry detergent or a dishwashing detergent.

3. The cleaning composition of claim 1, wherein the cleaning composition is a single composition comprising the lipolytic enzyme and the surfactant.

4. The cleaning composition of claim 1, wherein the cleaning composition is a two-part composition, the first part comprising the lipolytic enzyme and second part comprising the surfactant, wherein the first part and the second part are combined prior to contacting the stain.

5. The cleaning composition of claim 1, wherein the surfactant is a Triton or oxide non-ionic surfactant.

6. The cleaning composition of claim 1, wherein the surfactant is a zwitterionic surfactant.

7. The cleaning composition of claim 1, wherein the surfactant is a FOS-choline or sulfobetaine.

8. The cleaning composition of claim 1, wherein the surfactant has an aliphatic portion comprising at least 8 carbons.

9. A method for removing an oily stain from a surface, comprising:
contacting the surface with a lipolytic enzyme and a surfactant,
hydrolyzing fatty acid esters present in the oily stain with the lipolytic enzyme to produce free fatty acids, and
solubilizing the free fatty acids produced by the lipolytic enzyme with the surfactant,
thereby removing the oily stain from the surface.

10. The method of claim 9, wherein:
(a) the lipolytic enzyme and the surfactant are present in a single cleaning composition; or
(b) the lipolytic enzyme and the surfactant are present in different cleaning compositions that are combined prior to the contacting; or
(c) the lipolytic enzyme and the surfactant are present in different cleaning compositions that are combined upon the contacting.

11. The method of claim 9, wherein the method further includes rinsing the surface.

12. The method of any of claims 9 to 11, wherein the surfactant is a Triton or oxide non-ionic surfactant.

13. The method of any of claims 9 to 12, wherein the surfactant is a zwitterionic surfactant.

14. The method of any of claims 9 to 13, wherein the surfactant is a FOS-choline or sulfobetaine.

15. The method of any of claims 9 to 14, wherein the surface is a fabric surface, a dishware surface or a hard surface.
